(19) Europäisches Patentamt
European Patent Office
Office européen des brevets

(11) **EP 2 725 356 A2**

(12) **EUROPEAN PATENT APPLICATION**
published in accordance with Art. 153(4) EPC

(43) Date of publication:
**30.04.2014 Bulletin 2014/18**

(21) Application number: **12802701.8**

(22) Date of filing: **23.04.2012**

(51) Int Cl.:
***G01N 33/483*** (2006.01) ***G01N 21/64*** (2006.01)
***G01N 33/68*** (2006.01)

(86) International application number:
**PCT/KR2012/003126**

(87) International publication number:
**WO 2012/176977 (27.12.2012 Gazette 2012/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2011 KR 20110061410**
**23.04.2012 KR 20120042318**

(71) Applicant: **Korea Research Institute Of Bioscience And Biotechnology**
**Daejeon 305-806 (KR)**

(72) Inventors:
- **CHUNG, Sang Jeon**
  **Daejeon 305-806 (KR)**
- **KANG, Hyo Jin**
  **Daejeon 305-806 (KR)**
- **KIM, Ju Hwan**
  **Daejeon 305-806 (KR)**

(74) Representative: **Moore, Graeme Patrick**
**Mewburn Ellis LLP**
**33 Gutter Lane**
**London**
**EC2V 8AS (GB)**

(54) **MICROSCOPE APPARATUS FOR DETECTING OR IMAGING PROTEIN USING IFRET PROBE, AND METHOD FOR DETECTING OR IMAGING PROTEIN USING SAME**

(57) A microscope apparatus for detecting or imaging a target protein using a probe for intrinsic fluorescence resonance energy transfer (iFRET) according to the present invention comprises: a light irradiation unit that irradiates a first light having a wavelength range for exciting an amino acid in the target protein and a second light having a wavelength range for exciting a fluorescent molecule of the probe for iFRET; an objective lens that allows the lights irradiated from the light irradiation unit to be incident onto a sample into which the probe for iFRET is introduced; and a recognition unit that detects a first light emitting signal generated from the probe for iFRET by irradiating the first light having a wavelength range for exciting an amino acid in the target protein onto the sample and a second light emitting signal generated from the probe for iFRET by irradiating the second light having a wavelength range for exciting a fluorescent molecule of the probe for iFRET onto the sample, wherein the probe for iFRET includes: a binding site specific to a target protein or a molecule which has the binding site; and a fluorescent molecule having an acceptor function with respect to intrinsic fluorescence of the target protein, which are bonded to each other directly or by a linker.

FIG. 2

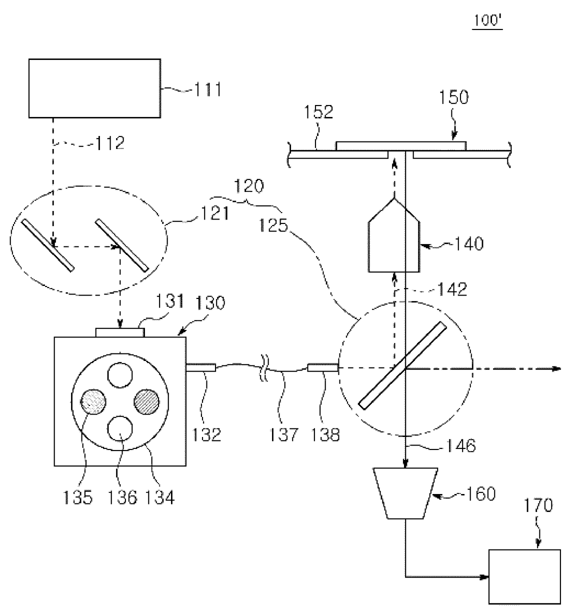


EP 2 725 356 A2

## Description

### [Technical Field]

The present invention relates to a microscope apparatus for detecting or imaging a protein using a probe for intrinsic fluorescence resonance energy transfer (iFRET) and a method for detecting or imaging a protein using the same.

### [Background Art]

Fluorescence microscopes using fluorescence have been widely used as optical equipment for observing microscopic samples in detail in various biomedical fields, including use for the fluorescence analysis of biochips such as genome chips. A fluorescence microscope is an apparatus configured to irradiate light onto a sample as a microscopic object and detect the fluorescence excited and emitted from the sample by the irradiated light to thereby observe information such as the image of the microscopic object. Typically, when a fluorescence microscope is used for measurement, various probes or reporters are inserted onto a sample to be measured.

Meanwhile, in many life science-related fields, various materials have been used as probes or reporters in *vivo* or *in vitro* for analyzing biomaterials. Such probe or reporter methods can be largely divided into methods for measuring coloring, color formation, and light emission caused by the decomposition or deformation of a specific substrate by itself or by an enzyme reaction, and methods for observing a material that has a fluorescent characteristic or a coloring characteristic.

The former methods have been applied to not only intracellular observation such as the measurement of an expressed amount of a protein by being fused into the protein, or the measurement of the intensity of a promoter, selection of a recombinant cell, etc., but also extracellular observation such as western/northern blotting, ELISA (enzyme linked immunosorbent assay), etc. In a representative example, an enzyme such as alkaline phosphatase, peroxidase, xanthine oxidase, etc., and a compound such as luminol, lucigenin, acridinium ester, biotin, fluorescein, dioxigenin, etc. as a substrate are used.

However, if they are expressed in cells, there is the danger of inhibiting growth by disturbing metabolic pathways *in vivo* or inducing apoptosis. Further, the a catalytic characteristics of the enzymes and low stability of the substrate used are frequent causes of experimental error, and since most of them are not biomaterials but rather chemical compounds, there is a possibility that they can be harmful to the cells, and thus it is difficult to continuously carry out observation *in vivo.* Accordingly, most of the above-described probes or reporters have been mainly used for probing *in vitro* rather than *in vivo.*

Therefore, in order to solve the above-mentioned problem, a reporter in which the protein itself serves as a probe has been actively studied. A representative example in which the protein itself serves as a probe includes a fluorescent protein having a characteristic in which energy emitted when a fluorophore activated by absorbing a light having a specific wavelength is returned to a normal state with a lower energy is expressed in the form of light. Representative examples include a green fluorescent protein (GFP) expressing green fluorescence derived from the jellyfish Aequorea Victoria, and a Discosoma red fluorescent protein (DsRed) derived from Discocosoma species.

"Fluorescent protein" refers to proteins that exhibit fluorescence by themselves without a substrate or a cofactor. Therefore, they do not cause metabolism disorders and interference in cell physiological factors as much as the former case, and thus can be applied to various probe fields using the enzymes of the former methods and other fields, i.e. fields to which enzymes and substrates are difficult to be applied due to shortcomings thereof. In particular, while a protein is kept alive maintaining normal physiology without destruction of cells or organisms with fluorescence having an intrinsic wavelength generated by a protein in response to a light having a specific wavelength, such fluorescent proteins can directly monitor movement and active path of a specific protein and complicated intracellular changes such as cell division, cell differentiation, and the like, and have been recognized as elements superior to any other conventional methods.

Further, when a combination of a wild-type fluorescent protein with a red RFP, a yellow YFP, a blue green CFP, and a blue BFP improved to emit a light having a wavelength different from each other is adopted by using protein engineering technology, various phenomena can be observed at the same time. By using a FRET phenomenon occurring when the fluorescent proteins emitting a light having a wavelength different from each other are expressed as being adjacent to each other or fused into each other, it can be applied in various ways to be suitable for the purposes of study, such as a search for whether a specific material is present in a sample, observation of interaction between proteins, and the like.

Meanwhile, fluorescence resonance energy transfer is a phenomenon that if absorbing energy from outside, an energy donor, a shorter wavelength dye, transfers excitation energy radiationlessly to an energy acceptor, a longer wavelength excitation dye, positioned in a certain distance (< 10 nm), resulting in emitting a longer wavelength light from the acceptor instead of a shorter wavelength light from the donor. Miyawaki et al. (Nature, 1997, 388: 882-887) analyzed

interaction between calmodulin and calmodulin-binding peptide by using the FRET principle, and developed a novel protein interaction analysis system. The dyes absorb lights having intrinsic wavelengths different from each other and excite, and when the energy is emitted in the form of light or heat, the dyes are reinstated in a ground state and each dye emits a light having a unique wavelength range. The fluorescence resonance energy transfer is a phenomenon that if two fluorescent dyes different from each other are positioned within a distance of 10 to 100 Å, a light emitted after a shorter wavelength dye is excited induces excitation of a longer wavelength excitation dye to emit fluorescence. That is, after two different fluorescent dyes are attached to the back of two proteins with which an interaction is desired to be studied, by detecting fluorescence caused by fluorescence resonance energy transfer occurring by an interaction between the two proteins when the two fluorescent dyes are close to each other within 10 to 100 Å, a protein interaction can be analyzed.

By way of example, a fluorescent protein excited in the form of light having a wavelength of 488 nm emits fluorescence having a wavelength of 520 nm, which stimulates other fluorescent proteins in a pair, resulting in emitting fluorescence having a wavelength of 630 nm. Therefore, by exciting proteins with light of 488 nm and detecting fluorescence having a wavelength of 630 nm, it is possible to analyze an interaction between the two proteins. This system is capable of analyzing a dynamic interaction between proteins in a live cell, but it is capable of detection only when two fluorescent proteins are positioned at a very short distance from each other. Further, in some cases, this system needs overexpression of proteins in order to detect a subtle change in fluorescent wavelength.

In order to solve the above-described problem, in recent years, a use of intrinsic fluorescence of proteins has been studied, and it is reported that tryptophan as one of amino acids can be applied to FRET (Angew. Chem. Int. Ed. 2006, 45, 4562-4588). Further, multimeric tryptophan biosensors are known, which comprise tryptophan-binding domains and fluorescent moieties that permit detection and measurement of fluorescence resonance energy transfer upon tryptophan binding (U.S. Patent Laid-open Publication No. 2008/0311047).

However, probes used for the currently-known FRET techniques using intrinsic fluorescence of a protein may measure unnecessary signals made by overlapping emission wavelengths of the protein and a fluorescent molecule, thereby hindering sensitivity and specificity of a measurement result.

Thus, the present inventors continuously researched to solve a fundamental problem of conventional widely-used probe systems. As a result, the present inventors observed new fluorescent molecules using intrinsic fluorescence of proteins and developed a probe system in which these fluorescent molecules are bonded to a target protein binding site and also developed a microscope apparatus for detecting or imaging a protein using a probe for intrinsic fluorescence resonance energy transfer (iFRET) and a method for detecting or imaging a protein using the same, thereby completing the present invention.

**[Disclosure]**

**[Technical Problem]**

An object of the present invention is to provide a microscope apparatus for detecting or imaging a target protein using a probe for intrinsic fluorescence resonance energy transfer (iFRET); and a method for detecting or imaging a target protein using the microscope apparatus of the present invention.

**[Technical Solution]**

In order to achieve the above object, an exemplary embodiment of the present invention provides a microscope apparatus for detecting or imaging a target protein using a probe for intrinsic fluorescence resonance energy transfer (iFRET). The probe for iFRET includes: a binding site specific to a target protein or a molecule which has the binding site; and a fluorescent molecule having an acceptor function with respect to intrinsic fluorescence of the target protein, which are bonded to each other directly or by a linker, and the microscope apparatus comprises: a light irradiation unit that irradiates a first light having a wavelength range for exciting an amino acid in the target protein and a second light having a wavelength range for exciting a fluorescent molecule of the probe for iFRET; an objective lens that allows the lights irradiated from the light irradiation unit to be incident onto a sample into which the probe for iFRET is introduced; and a recognition unit that detects a first light emitting signal generated from the probe for iFRET by irradiating the first light having a wavelength range for exciting an amino acid in the target protein onto the sample and a second light emitting signal generated from the probe for iFRET by irradiating the second light having a wavelength range for exciting a fluorescent molecule of the probe for iFRET.

Preferably, the microscope apparatus further comprises: a ratiometric measurement module that analyzes a first light emitting signal value generated from the probe for iFRET by irradiating the first light having a wavelength range for exciting an amino acid in the target protein onto the sample and a second light emitting signal value generated from the probe for iFRET by irradiating the second light having a wavelength range for exciting a fluorescent molecule of the

probe for iFRET to calculate a third light emitting signal value by ratiometric measurement of the first light emitting signal value and the second light emitting signal value.

Further, an exemplary embodiment of the present invention provides a method for detecting or imaging a target protein using the microscope apparatus of the present invention, the method comprising: a first step of introducing the probe for iFRET into a sample containing the target protein; a second step of alternately irradiating a first light having a wavelength range for exciting an amino acid in the target protein and a second light having a wavelength range for exciting a fluorescent molecule of the probe for iFRET onto the sample prepared in the first step; and a third step of calculating a third light emitting signal value by analyzing a first light emitting signal value generated from the probe for iFRET by irradiating the first light having a wavelength range for exciting an amino acid in the target protein onto the sample and a second light emitting signal value generated from the probe for iFRET by irradiating the second light having a wavelength range for exciting a fluorescent molecule of the probe for iFRET to calculate the third light emitting signal value by ratiometric measurement of the first light emitting signal value and the second light emitting signal value.

**[Advantageous Effects]**

According to the present invention, if a probe for iFRET is used, an amino acid in a protein is used as a fluorescence donor unlike the conventional FRET technique. Therefore, an artificial marker (fluorescent protein, etc.) is not needed for a target protein and a single fluorescent material is enough. Further, the probe for iFRET has an emission wavelength separated from that of a protein intrinsic fluorescence and thus has high specificity and sensitivity. Therefore, the probe for iFRET can analyze amounts, activities, and mechanisms of various proteins more easily and more accurately and can also be used for diagnosis of a disease related to the target protein and development of a medicine therefor.

Therefore, if the microscope apparatus according to the present invention is used, it is easy to carry out a method for detecting or imaging a target protein bonded to the probe for iFRET; and by the method for detecting or imaging a target protein according to the present invention, it is possible to check the amount of the target protein, the activity of the target protein, and the action mechanism of the target protein or possible to trace a pathway of the target protein.

Further, by irradiating a first light having a wavelength range for exciting an amino acid in the target protein and a second light having a wavelength range for exciting a fluorescent molecule of the probe for iFRET onto a sample containing the target protein, it is possible to carry out an accurate quantitative analysis on the target protein; and if the first light and the second light are alternately irradiated onto the sample at regular intervals, it is possible to observe a behavior of the target protein via video.

**[Description of Drawings]**

FIG. 1 is a configuration view of a microscope apparatus for detecting or imaging a target protein using a probe for intrinsic fluorescence resonance energy transfer (iFRET) according to a first exemplary embodiment of the present invention;

FIG. 2 is a configuration view of a microscope apparatus according to a second exemplary embodiment of the present invention;

FIG. 3 is a configuration view of a microscope apparatus according to a third exemplary embodiment of the present invention;

FIG. 4 is a diagram illustrating photo-excitation and emission processes using a probe for iFRET included in a sample used in the present invention; and

FIG. 5 is a flow chart time-sequentially illustrating a detecting or imaging process of a target protein using a microscope apparatus of the present invention.

**[Best Mode]**

The above-described object, feature, and other advantages of the present invention will be more apparent from detailed explanation of exemplary embodiments of the present invention with reference to the accompanying drawings. The exemplary embodiments to be described will be provided only for illustrating the present invention and do not limit the scope of the present invention.

Respective components constituting a microscope apparatus for detecting or imaging a protein using a probe for iFRET may be manufactured in an integrated manner or in a separated manner as necessary. Further, some components may be omitted in actual implementation.

In the present invention, the term "FRET" refers to non-radiative energy transfer between two fluorescent materials having different emission wavelengths, in which the excitation energy of a fluorescence donor in an excited state is transferred to a fluorescence receiver, and thus emission from the fluorescence acceptor, or the quenching of the fluorescence donor is observed (Lakowicz, J.R. Principles of Fluorescence Spectroscopy, 2nd ed., New York: Plenum Press, 1999).

Generally, FRET is named because the emission wavelength of the fluorescence donor overlaps with the absorption spectrum of the fluorescence acceptor and FRET occurs without the appearance of a photon and FRET results from the long-range dipole interaction between the fluorescence donor and the fluorescence acceptor. The energy transfer efficiency of FRET varies depending on the range in which the emission spectrum of the fluorescence donor and the absorption spectrum of the fluorescence acceptor overlap with each other, the quantum efficiency of the fluorescence donor, the relative orientation of transition dipoles of the fluorescence donor and the fluorescence acceptor, and the distance between the fluorescence donor and the fluorescence acceptor. Thus, the energy transfer efficiency of FRET varies depending on the distance between the fluorescence donor and the fluorescence acceptor and the relative orientation thereof and is expressed as follows according to the Forster's equation.

$$E=R_0^6/(R^6+R_0^6) \quad \text{[Equation 1]}$$

In the above equation, E denotes FRET efficiency, and R denotes the distance between the fluorescence donor and the fluorescence acceptor and is generally 2 to 9 nm depending on the kind of a fluorescent material. Further, $R_0$ denotes the distance between the fluorescence donor and the fluorescence acceptor, at which FRET efficiency is 50%, and it is generally called "Forster distance" or "Forster radius". $R_0$ is expressed as the following equation.

$$R_0=0.211[k^2n^{-4}Q_DJ(\lambda)]^{1/6} \text{ (in Å)} \quad \text{[Equation 2]}$$

In the above equation, $k^2$ is an orientation factor that is usually calculated as 2/3, and has a value in the range of from 0 to 4 depending on the relative orientation of emission of the fluorescence donor and absorption of the fluorescence acceptor. Further, n is the refractive index of a medium and is usually ~1.334 for water at 25°C, and $Q_D$ is the quantum efficiency of the fluorescence donor. J(λ) is the degree of overlap between the emission spectrum of the fluorescence donor and the absorption spectrum of the fluorescence acceptor and is expressed in the unit of $M^{-1}\,cm^{-1}nm^4$ (Lakowicz, J.R. Principles of Fluorescence Spectroscopy, 2nd ed., New York: Plenum Press, 1999; Patterson et al., Anal. Biochem. 284: 438, 2000; Patterson et al., J. of Cell Sci. 114: 837, 2001).

In the present invention, the term "fluorescence donor" refers to an amino acid involved in exhibition of intrinsic fluorescence of a protein. Most proteins exhibit intrinsic fluorescence. It is known that amino acids having an aromatic ring structure are involved at this time. Examples of the amino acids may include tryptophan, tyrosine, and phenylalanine. It is known that these tryptophan, tyrosine, and phenylalanine are excited by a light having a wavelength of 260 to 300 nm and emit a light having a wavelength of 290 to 400 nm and this light has an influence on a wavelength of up to 440 nm.

In the present invention, the terms of "fluorescence receiver" and "fluorescence acceptor" are used similarly in meaning and it refers to a fluorescent molecule excited by intrinsic fluorescence of a protein and emitting a light.

A probe 155 for iFRET according to the present invention includes a binding site specific to a target protein or a molecule 156 (hereinafter, referred to as "binding molecule") that has the binding site, and a fluorescent molecule 158 having an iFRET acceptor function, and the binding molecule and the fluorescent molecule are bonded to each other directly or by a linker. The binding molecule site induces the probe for iFRET to make a bond specific to the target protein (see FIG. 3).

Preferably, the fluorescent molecule 158 having an acceptor function with respect to intrinsic fluorescence of the target protein according to the present invention may be excited by a light having a wavelength of 300 to 400 nm which is an emission wavelength of tryptophan, tyrosine, and/or phenylalanine as amino acids exhibiting the intrinsic fluorescence of the target protein, and more preferably, the fluorescent molecule 158 may have an emission wavelength of 450 nm or more which is a wavelength separated from that of a protein intrinsic fluorescence.

When a light emitted from the probe for iFRET is checked or traced, all wavelength ranges of lights emitted

from the probe for iFRET may be measured. However, preferably, among the lights emitted from the probe for iFRET, a light having a long wavelength of 450 nm or more may be measured. A cell or a tissue includes a nucleic acid as a cellular autofluorescent material that exhibits fluorescence in response to a light of having a wavelength of 260 to 400 nm, nucleic acid, NAD(P)H or collagen, or a cell protein. Fluorescence generated by them has an emission wavelength mainly in the range of 300 to 450 nm. Therefore, if the probe for iFRET of the present invention is used for imaging or used for staining a cell extract or tissue, in order to avoid interference therebetween, preferably, the probe for iFRET of the present invention has an emission region of 450 nm or more.

For example, if 1-naphthyldiamine as expressed as the following Chemical Formula I as the fluorescent molecule 158 having an acceptor function with respect to intrinsic fluorescence of the target protein in the probe for iFRET used in the present invention, it can be detected effectively by LED (light-emitting diode) and can be used as a fluorescent probe. Further, it has an excitation wavelength of 340 nm to 380 nm and an emission wavelength of 400 nm to 600 nm and thus has a higher emission wavelength range as compared with the well-known fluorescent material. Therefore, it can be measured separately from the cellular autofluorescent material.

[Chemical Formula I]

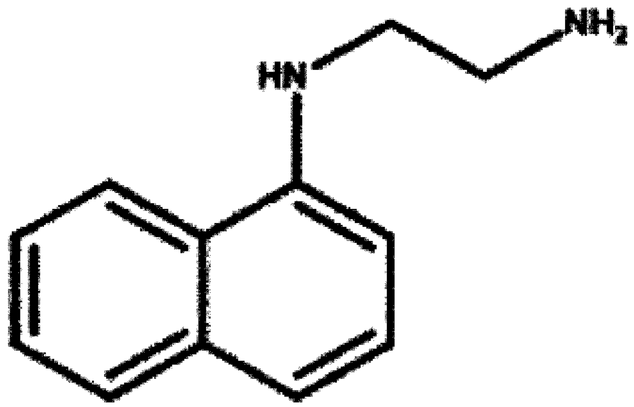

Meanwhile, in the present invention, the term "sample" refers to an observation target that contains a target protein or is suspected to contain the target protein and is to be analyzed, and it may be collected from one or more of the target protein itself, cells, blood, urine, water, soil, air, foods, waste, animal and plant organs and tissues, and an organism itself, but is not limited thereto.

If a target protein is processed with the probe for iFRET of the present invention, in order to check the probe for iFRET that emits a light by irradiating a light which excites an amino acid in the target protein, typical fluorescent material processing conditions may be used. Preferably, the process may be carried out in a medium selected from the group consisting of water, a buffer solution, low-grade alcohol having 1 to 6 carbon atoms, and mixtures thereof, or on a biochip or (ultra) fine particle, or to a cell or a tissue.

Hereinafter, a microscope apparatus for detecting or imaging a target protein and a method for detecting or imaging a target protein using the same according to exemplary embodiments of the present invention will be explained in detail with reference to the accompanying drawings.

**Explanation of overall configuration of microscope apparatus 100 for detecting or imaging target protein**

Referring to FIG. 1, a microscope apparatus 100 according to an exemplary embodiment of the present invention includes: a light irradiation unit 110 that irradiates a first light having a wavelength range for exciting an amino acid in the target protein and a second light having a wavelength range for exciting a fluorescent molecule of the probe for iFRET; an objective lens 140 that allows the lights irradiated from the light irradiation unit 110 to be incident onto a sample 150 into which the probe for iFRET is introduced; and a recognition unit 160 that detects a first light emitting signal generated from the probe 155 for iFRET by irradiating the first light having a wavelength range for exciting an amino acid in the target protein onto the sample and a second light emitting signal generated from the probe 155 for iFRET by irradiating the second light having a wavelength range for exciting a fluorescent molecule of the probe for iFRET onto the sample. A stage 152 on which the sample 150 is placed moves in three directions, i.e. x, y, and z, directions and enables the sample 150 to move three-dimensionally.

Preferably, the microscope apparatus of the present invention may further include a ratiometric measurement

module 170 that analyzes a first light emitting signal value generated from the probe for iFRET by irradiating the first light having a wavelength range for exciting an amino acid in the target protein onto the sample and a second light emitting signal value generated from the probe for iFRET by irradiating the second light having a wavelength range for exciting a fluorescent molecule of the probe for iFRET onto the sample to calculate a third light emitting signal value by ratiometric measurement of the first light emitting signal value and the second light emitting signal value. The ratiometric measurement module 170 may include a program for operating the module and a program for calculation.

A first light 142 having a wavelength range for exciting an amino acid in the target protein and a second light 142 having a wavelength range for exciting a fluorescent molecule of the probe for iFRET, which are generated from the light irradiation unit 110, may be concentrated at the objective lens 140 and transferred to the sample 150. In the sample 150, a first emitted light 146 generated from the probe 155 for iFRET by irradiating the first light having a wavelength range for exciting an amino acid in the target protein onto the sample and a second emitted light 146 generated from the probe 155 for iFRET by irradiating the second light having a wavelength range for exciting a fluorescent molecule of the probe for iFRET onto the sample are recognized by the recognition unit 160.

Typically, the sample 150 may contain a target protein 154 and the probe 155 for iFRET bonded to the target protein. The probe 155 for iFRET includes the binding molecule 156 which is specific to the target protein and the fluorescent molecule 158 having an iFRET acceptor function, which are bonded to each other directly or by a linker. The binding molecule site induces the probe for iFRET to make a bond specific to the target protein, whereby the target protein can be quantitated through iFRET, and also induces the probe for iFRET to move together with the target protein, whereby a behavior of the target protein can be checked.

Therefore, the first light emitting signal value, the second light emitting signal value, and the third light emitting signal value may be two-dimensional image values respectively corresponding to two dimensional points of the sample.

Meanwhile, the present invention is characterized by alternately irradiating a first light 142a having a wavelength range for exciting an amino acid in the target protein and a second light 142b having a wavelength range for exciting a fluorescent molecule of the probe for iFRET onto the sample that contains the target protein and the probe for iFRET bonded thereto.

The second light 142b having a wavelength range for exciting a fluorescent molecule of the probe for iFRET is a light that does not excite an amino acid in the target object but directly excites a fluorescent molecule of the probe for iFRET.

The ratiometric measurement module 170 can analyzes the first light emitting signal value generated from the probe for iFRET by irradiating the first light having a wavelength range for exciting an amino acid in the target protein onto the sample and the second light emitting signal value generated from the probe for iFRET by irradiating the second light having a wavelength range for exciting a fluorescent molecule of the probe for iFRET onto the sample to calculate the third light emitting signal value by ratiometric measurement of the first light emitting signal value and the second light emitting signal value.

The third light emitting signal value is a ratio of an amount (first light emitting signal value) of probes for iFRET bonded to the target protein to an amount (second light emitting signal value) of probes for iFRET present in the measurement sample (for example, a cell, a test liquid, or the like). Since a reference value (second light emitting signal value) is offered regardless of a temporal and spatial change to measurement, the third light emitting signal value enables quantification of bonds between the target protein and the probes for iFRET. Further, if ratiometric measurement is adopted, it is possible to make a correction even if an amount of lights irradiated to the sample is changed.

The third light emitting signal value can be calculated by the following equation.

Third light emitting signal value = First light emitting signal value ÷ Second light emitting signal value

[Equation 3]

By converting an intensity of the first light emitting signal value into the third light emitting signal value by using the second light emitting signal value as a reference according to ratiometric measurement, it is possible to minimize a measurement error caused by localization of the probes for iFRET and also possible to obtain clear images. Therefore, it is possible to quantitatively measure an amount of the target protein and bonds of medicine to the target protein, and also possible to trace a spatial movement of the target protein.

The third light emitting signal value can be calculated by using the conventional ratiometric measurement

software.

In the light irradiation unit 110, preferably, the first light having a wavelength range for exciting an amino acid in the target protein may have a wavelength in the range of from 260 nm to 300 nm, and the second light having a wavelength range for exciting a fluorescent molecule of the probe for iFRET may have a wavelength in the range of from 300 nm to 400 nm.

The light irradiation unit 110 may be a laser power control device which is capable of alternately irradiating the first light having a wavelength range for exciting an amino acid in the target protein and the second light having a wavelength range for exciting a fluorescent molecule of the probe for iFRET.

A microscope apparatus 100' according to a second exemplary embodiment of the present invention will be explained as follows with reference to FIG. 2. Hereinafter, only different parts from the first exemplary embodiment 100 will be explained, and the identical parts will be omitted.

As depicted in FIG. 2, the light irradiation unit 110 may include: a light source 111; and an excitation filter module 130 that selectively filters the first having a wavelength range for exciting an amino acid in the target protein and a second light having a wavelength range for exciting a fluorescent molecule of the probe for iFRET from the light source.

The light source may be a Xe/Hg (xenon/mercury) lamp. A metal halide lamp using mercury vapor and a xenon gas can improve lamp efficiency and can also reduce power consumption to 20% or less.

The light irradiation unit 110 may further include at least one first collimate mirror 121 that guides part of a light from the light source to the excitation filter module through reflection. The first collimate mirror 121 transmits a light having a longer wavelength than a first specific wavelength value (> 600 nm) and reflects a light having a shorter wavelength than the first specific wavelength value. Thus, it is possible to prevent a filter from being damaged by blocking excessive heat transfer from the light source to the filter, and the first collimate mirror 121 can be substituted with an infrared absorbing filter or the like. The first specific wavelength value has a wavelength range longer than that of the second light having a wavelength range for exciting a fluorescent molecule of the probe for iFRET. From a light having a wide range of wavelengths and generated from the light source 111, the first collimate mirror 121 first removes a wavelength range which does not need to be applied to the present invention. That is, the lights having a wavelength band of 600 nm or less are transmitted to the excitation filter module 130.

The excitation filter module 130 includes a photoresolution plate 134 in which multiple optical windows 135 and 136 are provided, a light input unit 131 that receives a reflected light from the first collimate mirror 121, and a light output unit 132 that sends a light.

The excitation filter module 130 can selectively transmit the first light 142a having a wavelength range for exciting an amino acid in the target protein and the second light 142b having a wavelength range for exciting a fluorescent molecule of the probe for iFRET. By performing a rotational operation to the photoresolution plate 134 periodically, the first optical window 135 that transmits the first light having a wavelength range for exciting an amino acid in the target protein and the second optical window 136 that transmits the second light having a wavelength range for exciting a fluorescent molecule of the probe for iFRET can be arranged on an optical path that connects the light input unit 131 and the light output unit 132. A light component output from the light output unit 132 may be discharged through an optical nozzle 138 via an optical interconnection line 137. The optical interconnection line 137 is made of a flexible material and thus makes it possible to freely select a position of the optical nozzle 138. Otherwise, the light output unit 132 and a second collimate mirror may be arranged in parallel with each other by an appropriate method.

The objective lens 140 concentrates light emitted from the sample 150 to produce an image. The objective lens 140 may be made of, for example, quartz or fused silica.

As depicted in FIG. 2, a second collimate mirror 125 may be provided on an optical path that connects the light irradiation unit 110 to the objective lens 140. In this case, the second collimate mirror 125 transmits a light having a longer wavelength than a second specific wavelength value and reflects a light having a shorter wavelength than the second specific wavelength value, and the second specific wavelength value has a wavelength range longer than that of the second light having a wavelength range for exciting a fluorescent molecule of the probe for iFRET and shorter than that of an emitted light, to be detected, generated from the probe for iFRET. Preferably, the second specific wavelength value may be 400 nm. This is because an absorption wavelength of the protein is in the range of 260 to 300 nm, an absorption wavelength of the probe for iFRET is in the range of 300 to 400 nm, and a final emission wavelength of the probe for iFRET is 400 nm or more.

The second collimate mirror 125 reflects the first light having a wavelength range for exciting an amino acid in the target protein, the second light having a wavelength range for exciting a fluorescent molecule of the probe for iFRET, or both of the lights and allows the first light or the second light to be incident to the sample through the objective lens 140. Meanwhile, the second collimate mirror 125 transmits the first emitted light generated from the probe for iFRET and derived from the first light irradiated onto the sample and the second emitted light generated from the probe for iFRET and derived from the second light irradiated onto the sample and allows the first emitted light and the second emitted light to be incident to the recognition unit 160 and to be detected as a first light emitting signal and a second light emitting signal, respectively.

Meanwhile, when the light irradiation unit alternately irradiates the first light having a wavelength range for exciting an amino acid in the target protein and the second light having a wavelength range for exciting a fluorescent molecule of the probe for iFRET at regular intervals, the third light emitting signal values ($C_1$, $C_2$, $C_3$,...) are calculated and accumulated by the ratiometric measurement module at regular intervals by ratiometric measurement of the first light emitting signal values ($A_1$, $A_2$, $A_3$,...) generated from the probe for iFRET by irradiating the first light having a wavelength range for exciting an amino acid in the target protein onto the sample and the second light emitting signal values ($B_1$, $B_2$, $B_3$,...) generated from the probe for iFRET by irradiating the second light having a wavelength range for exciting a fluorescent molecule of the probe for iFRET onto the sample, and, thus, a change in position, amount, or both of the target protein with time can be checked. Therefore, a change of the target protein with time can be calculated via video. Accordingly, an intracellular position, a spatial movement, a quantitative change, a change in activity, a binding degree of medicine, or all of the changes of the target protein with time can be checked.

Besides, a technique used for the typical fluorescence spectroscopy, for example, a technique used for a filter-type fluorescence spectroscopy and a monochrome-type fluorescence spectroscopy, may be employed.

A microscope apparatus 100" according to a third exemplary embodiment of the present invention will be explained as follows with reference to FIG. 3. Hereinafter, only different parts from the second exemplary embodiment 100' will be explained, and the identical parts will be omitted.

The present exemplary embodiment may include a first dichroic mirror 126 that guides part of a light from the light source 111 to the excitation filter module 130 through reflection. In the same manner as the first collimate mirror 121, the first dichroic mirror 126 transmits a light having a longer wavelength than the first specific wavelength value (> 600 nm) and reflects a light having a shorter wavelength than the first specific wavelength value, thereby first removing a wavelength range which does not need to be applied to the present invention from a light having a wide range of wavelengths.

A light component output from the light output unit 132 of the excitation filter module 130 passes through a relay lens system 144, a second dichroic mirror 146, and an objective lens 140', and a third dichroic mirror 148 and is detected by the recognition unit 160.

The relay lens system 144 allows a light filtered through the excitation filter module 130 to be concentrated at the second dichroic mirror 146 or the objective lens 140'. The second dichroic mirror 146 reflects an incident light having all wavelengths.

The objective lens 140' includes a first objective lens 141 positioned at a front end of the sample 150 and a second objective lens 145 positioned at a rear end of the sample 150. The first objective lens 141 may be a quartz or reflecting objective lens, and allows light to be concentrated onto the sample 150 for easy excitation.

Meanwhile, the second objective lens 145 can transmit a light of 340 nm or more among light components emitted from the sample 150 and can also magnify the sample 150. Preferably, the second objective lens 145 may be made of fused silica.

The third dichroic mirror 148 filters and removes a light having a wavelength of 310 nm or less. By removing an unnecessary light component which may remain while being supplied from the light source 111, an afterimage at the recognition unit 160 can be minimized.

Meanwhile, before a light passing through the third dichroic mirror 148 reaches the recognition unit 160, it may pass through a separate emission filter 149.

## Explanation of method for imaging target protein by using microscope apparatus 100

Hereinafter, a method for imaging a target protein by using a microscope apparatus according to an exemplary embodiment of the present invention will be explained with reference to FIGS. 2, 4, and 5.

First, the light source 111 generates a light having all wavelengths including a visible light or an invisible light (S10).

The generated light passes through the first collimate mirror 121 and the excitation filter module 130 and is divided into the first light 142a having a wavelength range for exciting an amino acid in the target protein and the second light 142b having a wavelength range for exciting a fluorescent molecule of the probe for iFRET (S20).

The lights having wavelengths divided and selected through the filtering in step S20 are concentrated at the objective lens 140 (S30). In this case, resolution can be improved by adjusting to various magnifications.

A light component irradiated from the objective lens 140 onto the sample 150 is excited by the probe 155 for iFRET included in the sample 150 and emitted as a light having a wavelength in a visible light region (S40). Herein, the light that excites the probe 155 for iFRET may repeatedly alternate between the first light 142a having a wavelength range for exciting an amino acid in the target protein and the second light 142b having a wavelength range for exciting a fluorescent molecule of the probe for iFRET.

The recognition unit 160 detects a light emitting signal generated from the probe 155 for iFRET (S50). The recognition unit 160 may be a CCD camera module. The CCD camera module includes an array of concentrators in a

sealed space and converts a pattern of incident photon energy into a discrete analog signal.

By ratiometric measurement of the first light emitting signal value generated from the probe for iFRET by irradiating the first light having a wavelength range for exciting an amino acid in the target protein onto the sample and the second light emitting signal value generated from the probe for iFRET by irradiating the second light having a wavelength range for exciting a fluorescent molecule of the probe for iFRET onto the sample, the third light emitting signal value is calculated (S60).

Preferably, the imaging method of the present invention is carried out by an automated workstation.

Regarding the probe for iFRET, Korean Patent Application (Probe for iFRET and use thereof) of the present inventors field on the same date is incorporated herein by reference in its entirety.

The exemplary embodiments of the present invention have been explained, but the present invention is not limited to the above-described specific exemplary embodiments. That is, it shall be understood that various modifications and changes of the present invention can be made by one of ordinary skill in the art without departing from the concept and scope of the accompanying claims, and

their equivalents are included in the scope of the present invention.

## Claims

1. A microscope apparatus for detecting or imaging a target protein using a probe for intrinsic fluorescence resonance energy transfer (iFRET), wherein the probe for iFRET includes: a binding site specific to the target protein or a molecule which has the binding site; and a fluorescent molecule having an acceptor function with respect to intrinsic fluorescence of the target protein, which are bonded to each other directly or by a linker, and
the microscope apparatus comprises:

    a light irradiation unit that irradiates a first light having a wavelength range for exciting an amino acid in the target protein and a second light having a wavelength range for exciting a fluorescent molecule of the probe for iFRET;
    an objective lens that allows the lights irradiated from the light irradiation unit to be incident onto a sample into which the probe for iFRET is introduced; and
    a recognition unit that detects a first light emitting signal generated from the probe for iFRET by irradiating the first light having a wavelength range for exciting an amino acid in the target protein onto the sample and a second light emitting signal generated from the probe for iFRET by irradiating the second light having a wavelength range for exciting a fluorescent molecule of the probe for iFRET onto the sample.

2. The microscope apparatus of claim 1, further comprising:

    a ratiometric measurement module that analyzes a first light emitting signal value generated from the probe for iFRET by irradiating the first light having a wavelength range for exciting an amino acid in the target protein onto the sample and a second light emitting signal value generated from the probe for iFRET by irradiating the second light having a wavelength range for exciting a fluorescent molecule of the probe for iFRET onto the sample to calculate a third light emitting signal value by ratiometric measurement of the first light emitting signal value and the second light emitting signal value.

3. The microscope apparatus of claim 2, wherein the first light emitting signal value, the second light emitting signal value, and the third light emitting signal value are two-dimensional image values respectively corresponding to two dimensional points of the sample.

4. The microscope apparatus of claim 1, wherein the first light has a wavelength in the range of from 260 nm to 300 nm, and the second light has a wavelength in the range of from 300 nm to 400 nm.

5. The microscope apparatus of claim 1, wherein an amino acid which exhibits intrinsic fluorescence of the target protein is tryptophan, tyrosine, phenylalanine, or a combination thereof.

6. The microscope apparatus of claim 1, wherein the light irradiation unit comprises:

    a light source; and
    an excitation filter module that selectively filters the first light having a wavelength range for exciting an amino acid in the target protein and the second light having a wavelength range for exciting a fluorescent molecule of

the probe for iFRET from the light source.

**7.** The microscope apparatus of claim 6, further comprising:

at least one first collimate mirror that guides part of a light from the light source to the excitation filter module through reflection,
wherein the first collimate mirror transmits a light having a longer wavelength than a first specific wavelength value and reflects a light having a shorter wavelength than the first specific wavelength value, and
the first specific wavelength value has a wavelength range longer than that of the second light having a wavelength range for exciting a fluorescent molecule of the probe for iFRET.

**8.** The microscope apparatus of claim 6, wherein the objective lens comprises a first objective lens positioned at a front end of the sample and a second objective lens positioned at a rear end of the sample, and
the first objective lens is a quartz or reflecting objective lens, and the second objective lens is a fused silica objective lens.

**9.** The microscope apparatus of claim 1, further comprising a second collimate mirror provided on an optical path that connects the light irradiation unit to the objective lens, wherein the second collimate mirror transmits a light having a longer wavelength than a second specific wavelength value and reflects a light having a shorter wavelength than the second specific wavelength value, and
the second specific wavelength value has a wavelength range longer than that of the second light having a wavelength range for exciting a fluorescent molecule of the probe for iFRET and shorter than that of an emitted light, to be detected, generated from the probe for iFRET.

**10.** The microscope apparatus of claim 9, wherein the second collimate mirror reflects the first light having a wavelength range for exciting an amino acid in the target protein, the second light having a wavelength range for exciting a fluorescent molecule of the probe for iFRET, or both of the lights and allows the first light or the second light to be incident to the sample through the objective lens, and
the second collimate mirror transmits the first emitted light generated from the probe for iFRET and derived from the first light irradiated onto the sample and the second emitted light generated from the probe for iFRET and derived from the second light irradiated onto the sample and allows the first emitted light and the second emitted light to be incident to the recognition unit and to be detected as a first light emitting signal and a second light emitting signal, respectively.

**11.** The microscope apparatus of claim 2, wherein the light irradiation unit alternately irradiates the first light having a wavelength range for exciting an amino acid in the target protein and the second light having a wavelength range for exciting a fluorescent molecule of the probe for iFRET at regular intervals, and
the third light emitting signal value is calculated and accumulated by the ratiometric measurement module at regular intervals by ratiometric measurement of the first light emitting signal value generated from the probe for iFRET by irradiating the first light having a wavelength range for exciting an amino acid in the target protein onto the sample and the second light emitting signal value generated from the probe for iFRET by irradiating the second light having a wavelength range for exciting a fluorescent molecule of the probe for iFRET onto the sample, and, thus, a change in position, amount, or both of the target protein with time is checked.

**12.** The microscope apparatus of claim 11, wherein the change of the target protein with time is calculated via video.

**13.** The microscope apparatus of claim 1, wherein the sample is the target protein itself, a solution, cells, blood, urine, water, soil, air, foods, waste, animal and plant organs and tissues, and an organism itself.

**14.** A method for detecting or imaging a target protein using a microscope apparatus of any one of claims 1 to 13, the method comprising:

a first step of introducing the probe for iFRET into a sample containing the target protein;
a second step of alternately irradiating a first light having a wavelength range for exciting an amino acid in the target protein and a second light having a wavelength range for exciting a fluorescent molecule of the probe for iFRET onto the sample prepared in the first step; and
a third step of calculating a third light emitting signal value by analyzing a first light emitting signal value generated from the probe for iFRET by irradiating the first light having a wavelength range for exciting an amino acid in

the target protein onto the sample and a second light emitting signal value generated from the probe for iFRET by irradiating the second light having a wavelength range for exciting a fluorescent molecule of the probe for iFRET onto the sample to calculate the third light emitting signal value by ratiometric measurement of the first light emitting signal value and the second light emitting signal value.

**15.** The method of claim 14, wherein the first light having a wavelength range for exciting an amino acid in the target protein and the second light having a wavelength range for exciting a fluorescent molecule of the probe for iFRET are alternately irradiated at regular intervals, and
the third light emitting signal value is calculated and accumulated at regular intervals by ratiometric measurement of the first light emitting signal value generated from the probe for iFRET by irradiating the first light having a wavelength range for exciting an amino acid in the target protein onto the sample and the second light emitting signal value generated from the probe for iFRET by irradiating the second light having a wavelength range for exciting a fluorescent molecule of the probe for iFRET onto the sample, and, thus, a change in position, amount, or both of the target protein with time is checked.

**16.** The method of claim 15, wherein the change of the target protein with time is calculated via video.

FIG. 1

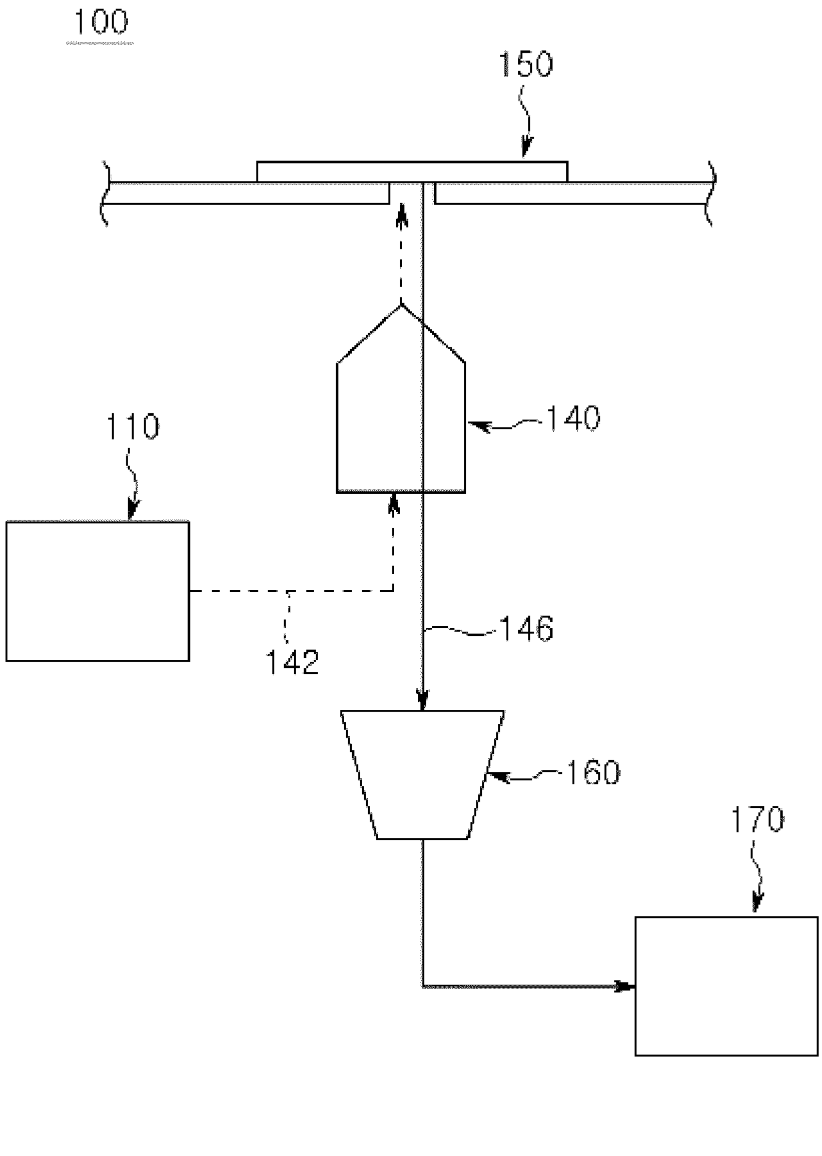

FIG. 2

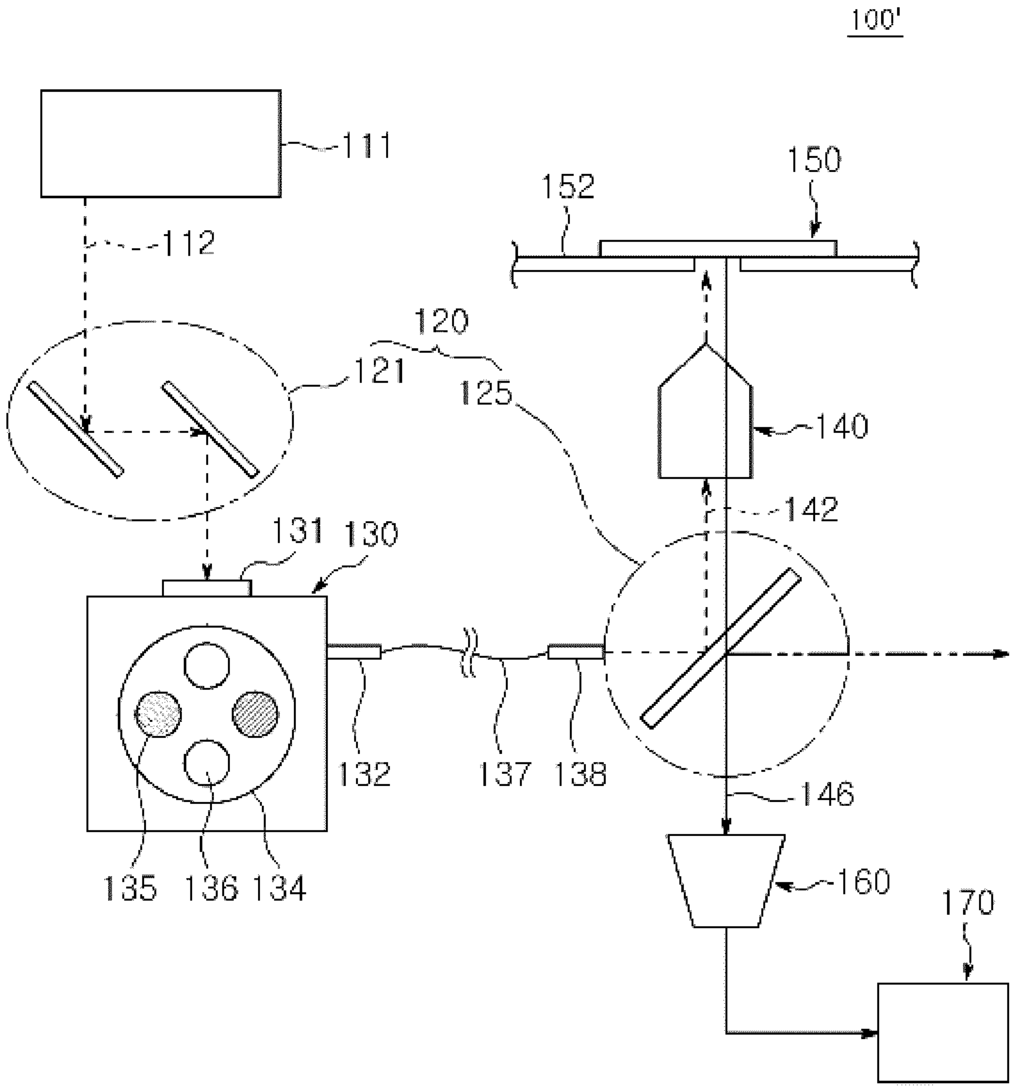
100'
111
112
120
121
125
131
130
132
137
138
135
136
134
152
150
140
142
146
160
170

FIG. 3

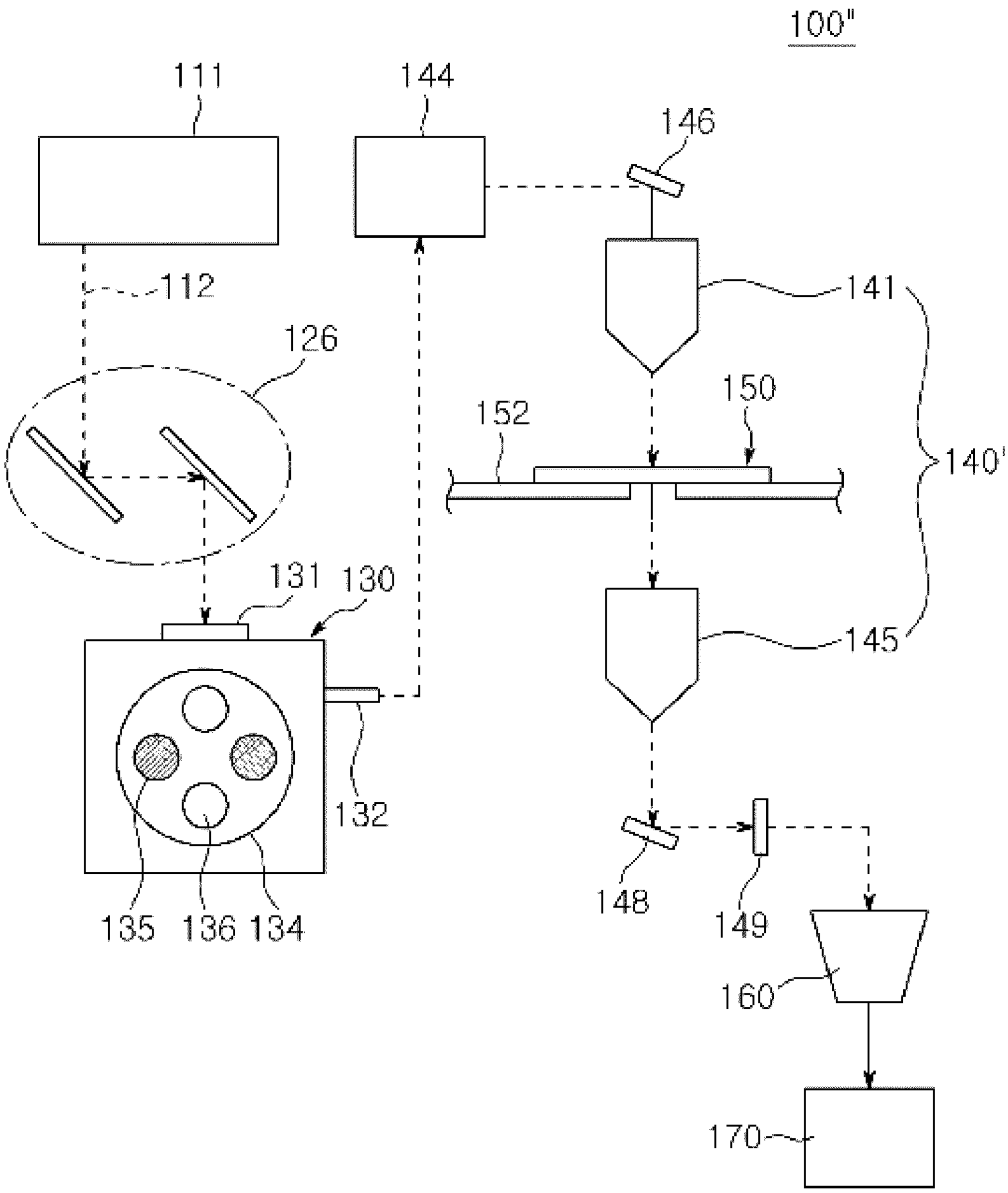
100"
111
144
146
112
141
126
150
152
140'
131
130
145
132
135
136
134
148
149
160
170

FIG. 4

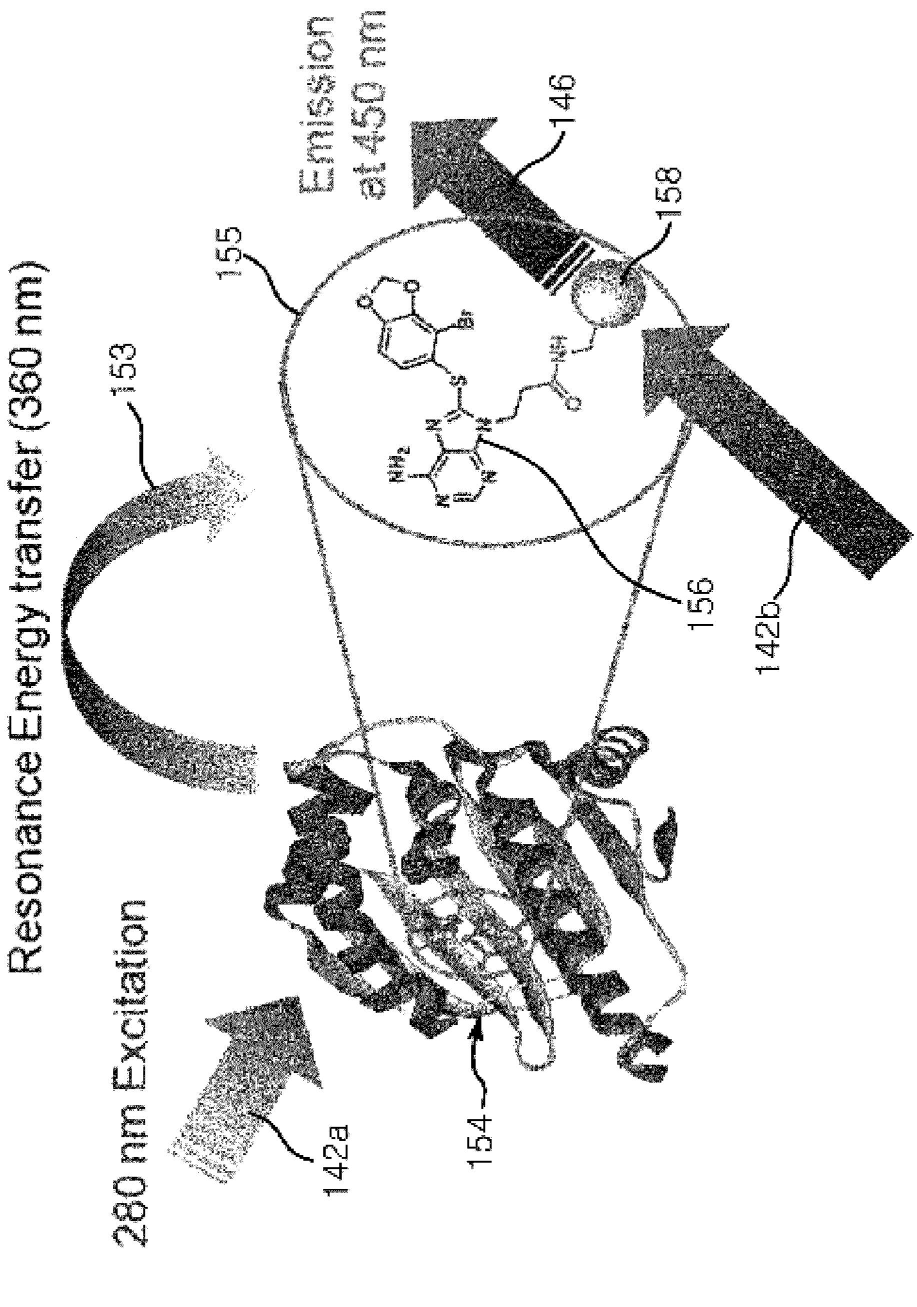
Resonance Energy transfer (360 nm)
280 nm Excitation
Emission at 450 nm
153
155
146
158
156
142b
142a
154

FIG. 5

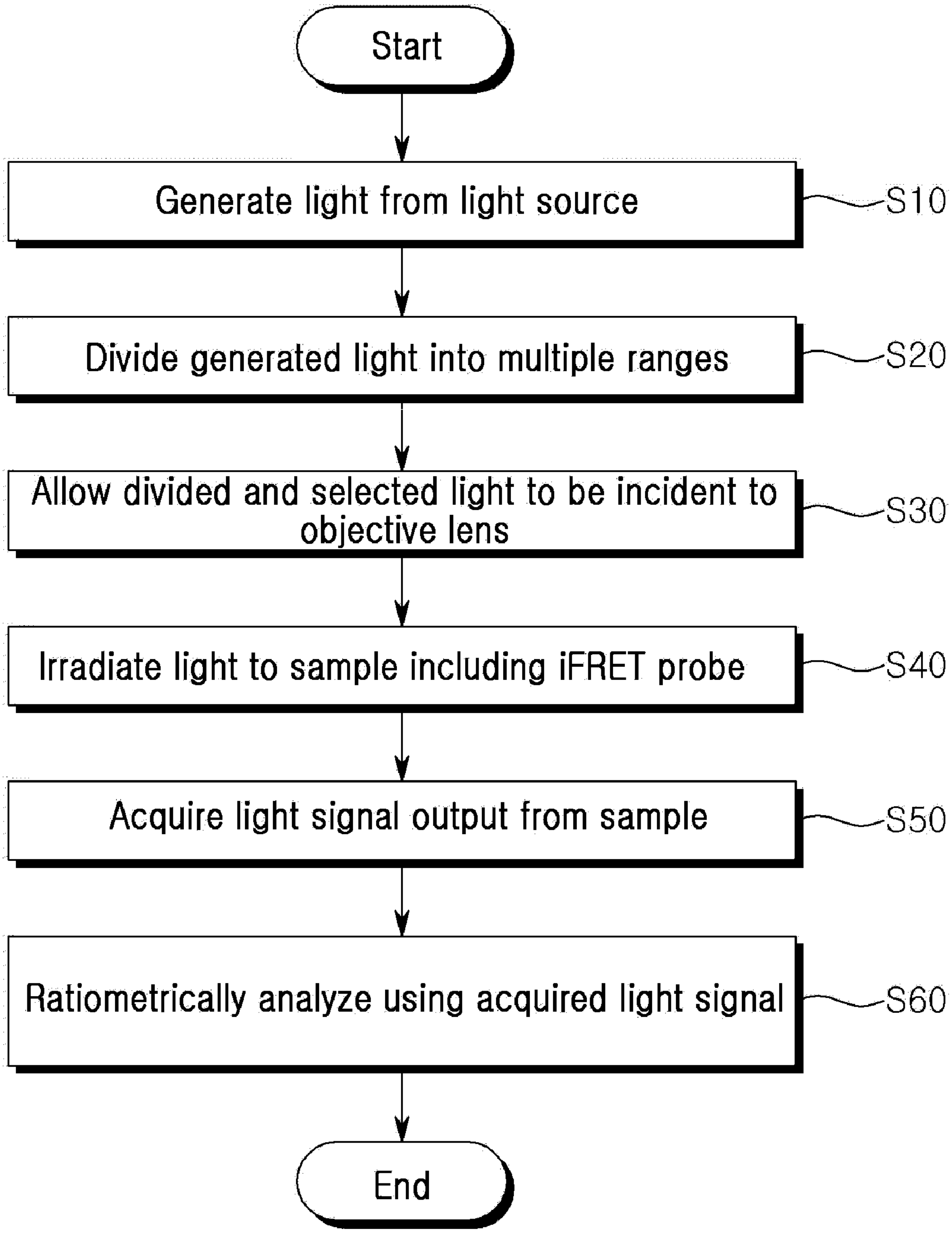
Start
Generate light from light source
S10
Divide generated light into multiple ranges
S20
Allow divided and selected light to be incident to objective lens
S30
Irradiate light to sample including iFRET probe
S40
Acquire light signal output from sample
S50
Ratiometrically analyze using acquired light signal
S60
End

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080311047 A


### Non-patent literature cited in the description


- **MIYAWAKI et al.** *Nature,* 1997, vol. 388, 882-887
- *Angew. Chem. Int. Ed.,* 2006, vol. 45, 4562-4588
- **LAKOWICZ, J.R.** Principles of Fluorescence Spectroscopy. Plenum Press, 1999
- **PATTERSON et al.** *Anal. Biochem.,* 2000, vol. 284, 438
- **PATTERSON et al.** *J. of Cell Sci.,* 2001, vol. 114, 837